(19) Europäisches Patentamt
European Patent Office
Office européen des brevets

(11) Veröffentlichungsnummer: **0 160 173 B1**

# (12) EUROPÄISCHE PATENTSCHRIFT

(45) Veröffentlichungstag der Patentschrift: **14.06.89**

(51) Int. Cl.⁴: **C 07 D 277/82**, A 61 K 31/425

(21) Anmeldenummer: **85102091.7**

(22) Anmeldetag: **26.02.85**

(54) **Benzothiazolderivate, Verfahren zu ihrer Herstellung und diese Verbindungen enthaltendes Arzneimittel.**

(30) Priorität: **12.04.84 DE 3413875**

(43) Veröffentlichungstag der Anmeldung: **06.11.85 Patentblatt 85/45**

(45) Bekanntmachung des Hinweises auf die Patenterteilung: **14.06.89 Patentblatt 89/24**

(84) Benannte Vertragsstaaten: **AT BE CH DE FR GB IT LI LU NL SE**

(56) Entgegenhaltungen:
**EP-A- 0 028 406**
**JP-A-76 035 428**
**US-A- 3 912 748**

(73) Patentinhaber: **LUDWIG HEUMANN & CO GMBH, Heideloffstrasse 18-28 Postfach 2260, D-8500 Nürnberg (DE)**

(72) Erfinder: **Schickaneder, Helmut, Dr. Dipl.-Chem., Moosäcker 25, D-8501 Eckenthal (DE)**
Erfinder: **Mörsdorf, Peter, Dr. Dipl.-Chem., Untere Bahnhofstrasse 16, D-8501 Cadolzburg (DE)**
Erfinder: **Engler, Heidrun, Dr., Ringstrasse 23, D-8501 Cadolzburg (DE)**
Erfinder: **Brune, Kay, Prof. Dr., Am Weiheracker 17, D-8525 Marloffstein (DE)**
Erfinder: **Szelenyi, Istvan, Dr., Brahmsstrasse 16, D-8501 Schwaig (DE)**
Erfinder: **Ahrens, Kurt Henning, Dr., Praterstrasse 9, D-8500 Nürnberg (DE)**

(74) Vertreter: **Kraus, Walter, Dr. et al, Patentanwälte Kraus, Weisert & Partner Thomas-Wimmer-Ring 15, D-8000 München 22 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Die Erfindung betrifft neue Benzothiazolderivate, ein Verfahren zu ihrer Herstellung, ihre Verwendung als Arzneimittel, insbesondere ihre Verwendung als Lipoxygenase- bzw. Cyclooxygenasehemmer, diese enthaltende Arzneimittel und deren Herstellung.

Man weiss heute, dass an der Entstehung von entzündlichen und allergischen Prozessen Arachidonsäuremetaboliten, wie zyklische Endoperoxide, Slow Reacting Substances of Anaphylaxis (SRS-A, Leukotriene), Prostaglandine und Thromboxane, beteiligt sind. Diese Metaboliten werden durch die Enzyme Lipoxygenase und Cyclooxygenase gebildet. Es ist daher vorteilhaft, Arzneimittel zu entwickeln, die Wirkstoffe enthalten, welche eine umfassendere antiphlogistische Wirkung entwickeln als die bis heute bekannten. Bis heute sind relativ wenige Verbindungen bekannt, die entweder selektiv die Lipoxygenase oder gleichzeitig Lipoxygenase und Cyclooxygenase hemmen. Bekannte Hemmer dieser Art sind zum Beispiel Benoxaprofen und 3-Amino-1-(3-trifluormethylphenyl)-pyrazolin (vgl. B. Samuelson, Angew. Chem. 94, 881 (1982), E.J. Goetzl, Immunology 40, 709, (1980) und C.L. Malusten, in P. Venge, Ed., «The inflammatory process», Almqvist & Wiksell International, Stockholm, S. 73 (1981)).

Die US-A 3 912 748 beschreibt 5- und 6-Benzoxazolylalkansäuren, die gegebenenfalls in 2-Position substituiert sind, sowie die Derivate davon. Nach den Angaben dieser Druckschrift besitzen diese Verbindungen eine entzündungshemmende, antipyretische und analgetische Aktivität.

Die JP-A 51/35 428 beschreibt bestimmte Benzothiazolderivate zur Verwendung als Germizide in Landwirtschaft und Gartenbau.

Der Erfindung liegt die Aufgabe zugrunde, neue Hemmstoffe für die Enzyme Cyclooxygenase und/oder Lipoxygenase mit verbesserter Wirksamkeit zur Verfügung zu stellen.

Diese Aufgabe wird durch die Erfindung gelöst. Gegenstand der Erfindung sind daher neue Benzothiazolderivate der allgemeinen Formel I

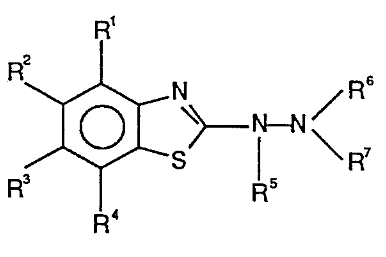

(I)

in der $R^1$, $R^2$, $R^3$ und $R^4$ jeweils ein Wasserstoffatom bedeuten, $R^5$ für ein Wasserstoffatom oder eine Methylgruppe steht, $R^6$ ein Wasserstoffatom und $R^7$ eine Formylgruppe bedeuten oder $R^6$ und $R^7$ für die Gruppierung stehen,

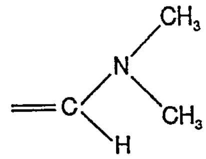

sowie die physiologisch verträglichen Salze davon.

Überraschenderweise hemmen die erfindungsgemässen Benzothiazolderivate in einem sehr starken Masse zum Teil selektiv das Enzym Lipoxygenase, und zwar sehr spezifisch bereits in solchen Konzentrationen, bei denen Cyclooxygenase nicht beinflusst wird, während einige Benzothiazolderivate beide Enzyme, Lipoxygenase und Cyclooxygenase, inhibieren. Die erfindungsgemässen cyclooxygenase- und/oder lipoxygenasehemmenden Benzothiazolderivate sind somit als Arzneimittel bei der Behandlung von entzündlichen und allergischen Erkrankungen einsetzbar.

Die erfindungsgemässen Benzothiazolderivate können durch ein Verfahren hergestellt werden, das dadurch gekennzeichnet ist, dass man

a) ein Benzothiazolderivat der allgemeinen Formel II

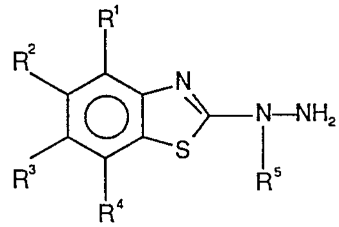

(II)

in der $R^1$ bis $R^5$ die oben angegebenen Bedeutungen besitzen, mit Ameisensäure zu einer erfindungsgemässen Verbindung der allgemeinen Formel I, in der $R^6$ ein Wasserstoffatom und $R^7$ eine Formylgruppe bedeutet, umsetzt oder

b) ein Benzothiazolderivat der allgemeinen Formel II

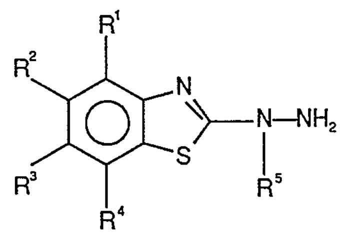

(II)

in der $R^1$ bis $R^5$ die oben angegebenen Bedeutungen besitzen, mit einem Dialkylaminoacetal der allgemeinen Formel VIII

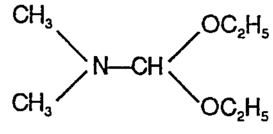

(VIII)

zu einer erfindungsgemässen Verbindung der allgemeinen Formel I, in der $R^6$ und $R^7$ für die Gruppierung stehen, umsetzt und

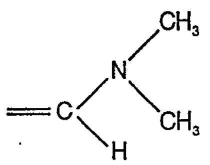

die erhaltenen Derivate gegebenenfalls in ihre physiologisch verträglichen Salze umwandelt.

Im Falle der Variante a) erfolgt die Umsetzung vorzugsweise mit wässriger Ameisensäure und bevorzugt im Molverhältnis 1:4 1/2 bis 1 Stunde bei 80 bis 100 °C, insbesondere 100 °C.

Im Falle der Variante b) erfolgt die Umsetzung mit dem Dialkylaminoacetal der allgemeinen Formel VIII über 3 bis 5 Stunden, vorzugsweise 2 Stunden, und bei einer Temperatur von 80 bis 120 °C, vorzugsweise bei 110 °C.

Die Isolierung der erfindungsgemässen Verbindungen sowie die Herstellung ihrer physiologisch annehmbaren Salze erfolgt in an sich bekannter Weise.

Die erfindungsgemässen Verbindungen können mit geeigneten Säuren in ihre physiologisch verträglichen Salze überführt werden. Diese Umsetzung erfolgt in bekannter Weise. Geeignete Säuren sind anorganische und organische Säuren. Beispiele für anorganische Säuren sind Salzsäure und Bromwasserstoffsäure. Beispiele für geeignete organische Säuren sind Oxalsäure, Apfelsäure und Bernsteinsäure. Grundsätzlich sind alle in der Pharmazie geeigneten anorganischen und organischen Säuren für die Umwandlung in das physiologisch verträgliche Salz einsetzbar.

Die erfindungsgemässen Verbindungen der allgemeinen Formel I bilden Säureadditionssalze, und es können tautomere Formen dieser Verbindung auftreten. Daher umfasst die vorliegende Erfindung auch die tautomeren Formen der Verbindungen der allgemeinen Formel I.

Die erfindungsgemässen Verbindungen, vorzugsweise in der Form eines Salzes, können zur Verabreichung in jeder beliebigen Weise formuliert werden. Die Erfindung umfasst daher auch Arzneimittel, die mindestens eine erfindungsgemässe Verbindung zur Verwendung in der Human- oder Veterinärmedizin enthalten. Solche Arzneimittel können herkömmlicherweise unter Verwendung eines oder mehrerer pharmazeutisch annehmbarer Träger oder Verdünnungsmittel hergestellt werden.

Die erfindungsgemässe Verbindung wird vorzugsweise oral verabreicht. Gewöhnlich beträgt die orale Tagesdosis etwa 0,05 bis 100 mg/kg, vorzugsweise 0,1 bis 10 mg/kg Körpergewicht. Im Einfall kann es gegebenenfalls erforderlich sein, von den genannten Mengen abzuweichen, und zwar in Abhängigkeit vom individuellen Verhalten gegenüber dem Wirkstoff bzw. der Art seiner Formulierung und dem Zeitpunkt bzw. Intervall, zu welchem die Verabreichung erfolgt. So gibt es zum Beispiel Fälle, wo mit weniger als der oben genannten Mindestmenge ausgekommen werden kann, während in anderen Fällen die genannte obere Grenze überschritten werden muss. Im Falle der Applikation grösserer Mengen kann es empfehlenswert sein, diese in mehreren Einzelgaben über den Tag zu verteilen.

Für die orale Verabreichung kann der Wirkstoff beispielsweise in Form von Kapseln formuliert werden, die durch herkömmliche Massnahmen mit pharmazeutisch annehmbaren Exzipientien, zum Beispiel Bindemitteln (wie vorgelatinisierter Maisstärke, Polyvinylpyrrolidon oder Hydroxypropylmethylcellulose), Füllstoffen (wie Lactose, mikrokristalline Cellulose oder Calciumphosphat), Schmiermitteln (wie Magnesiumstearat, Talk oder Kieselsäure), Sprengmitteln (zum Beispiel Kartoffelstärke oder Natriumstärkeglykollat) oder Befeuchtungsmitteln (zum Beispiel Natriumlaurylsulfat), hergestellt werden.

Flüssige Zubereitungen für die orale Verabreichung oder für ein direktes Einträufeln können beispielsweise die Form von Lösungen, Sirups oder Suspensionen haben, oder sie können als Trockenprodukt zur Rekonstitution mit Wasser oder einem anderen geeigneten Träger vor dem Gebrauch präsentiert werden. Solche flüssigen Zubereitungen können durch herkömmliche Massnahmen mit pharmazeutisch annehmbaren Additiven, zum Beispiel Suspendierungsmitteln, wie Sorbitsirup, Methylcellulose oder hydrierten essbaren Fetten, Emulgierungsmitteln, zum Beispiel Lecithin oder Acacia, nichtwässrigen Trägern, zum Beispiel Mandelöl, öligen Estern oder Ethylalkohol, und Konservierungsmitteln, zum Beispiel Methyl- oder Propylparahydroxybenzoaten oder Sorbinsäure, hergestellt werden.

Für die bukkale Verabreichung können die Zubereitungen in Form von Tabletten oder Lutschpastillen, die auf herkömmliche Weise formuliert werden, vorliegen.

Die erfindungsgemässen Verbindungen können für die parenterale Verabreichung durch Injektion oder für die Infusion formuliert werden. Zubereitungen für die Injektion können in Dosiseinheitsform, zum Beispiel in Ampullen oder in Mehrfachdosenbehältern mit einem zugegebenen Konservierungsstoff vorliegen.

Die Zubereitungen können auch solche Formen wie Suspensionen, Lösungen oder Emulsionen in öligen oder wässrigen Trägern einnehmen, sie können Formulierungsmittel, wie Suspendierungs-, Stabilisierungs- und/oder Dispergierungsmittel, enthalten.

Alternativ kann der Wirkstoff auch in Pulverform zur Rekonstitution mit einem geeigneten Träger, zum Beispiel sterilem, pyrogenfreiem Wasser, vor dem Gebrauch vorliegen.

Die folgenden pharmakologischen Untersuchungen der erfindungsgemässen Verbindungen geben Aufschluss über ihre überraschende antiphlogistische Wirksamkeit.

1. Rattenpfoten-Ödem-Test

1.1. Methode

Bei männlichen Ratten (100-120 g) wurde durch subplantare Injektion von 0,1 ml 2% Carrageenin (in wässriger 0,9%-NaCl-Lösung gelöst) ein akutes entzündliches Pfotenödem hervorgerufen. Das Pfotenvolumen wurde 1 bis 4 Stunden nach der Carrageenin-Injektion mittels eines Pfotenvolumenmessgeräts gemessen. Es wurden die Differenzen in den Pfotenvolumina zwischen den zwei Messungen berechnet. Die Prüfsubstanzen wurden in 1%iger Tylose suspendiert, eine Stunde nach der Carrageenin-Injektion intragastral mittels einer Magenschlundsonde verabreicht. Die Kontrolltiere erhielten nur den Trägerstoff (1% Tylose). Die prozentuale Hemmung der Volumenzunahme bei den behandelten Tieren ergab sich durch Vergleich mit den unbehandelten Kontrolltieren. Die mittleren Hemmdosen ($ED_{50}$) wurden mit Hilfe der Regressionskurven berechnet.

1.2. $ED_{50}$-Werte

Tabelle 1

| Beispiel Nr. | $ED_{50}$ (mg/kg) ig |
|---|---|
| 1 | 14 |
| 2 | 15 |
| 3 | 50 |

2. Leukozyten-Migrationstest

2.1. Methode

Bei männlichen Ratten (140-150 g) wurde ein mit 2%iger Carrageenin-Lösung getränktes Polyesterschwämmchen im Nackenbereich subkutan implantiert (vgl. Higgs, G.A. u.a., Eur. J. Pharmacol, 66, 81 (1980), Ford-Hutchinson, A.W. u.a., J. Pharmacol. Methods 1, 3 (1978)). 24 Stunden später wurden die Schwämmchen entfernt. Das Auswaschen der eingewanderten Leukozyten erfolgte in einer PBS-Lösung (pH 7,4, 0,5% Trypsin, 10 Einheiten/ml Heparin) bei 37°C 30 Minuten lang. Anschliessend wurden die Schwämmchen vorsichtig ausgedrückt und zentrifugiert (15 Minuten, 500 Upm). Die Leukozyten in der Waschflüssigkeit wurden dann ausgezählt. Die Prüfsubstanzen, die in 1%iger Tylose aufgenommen wurden, und das Vehikel (1%ige Tylose) wurden unmittelbar nach der Implantation des Schwämmchens, nach 5 und nach 21 Stunden später intragastral mittels einer Schlundsonde appliziert. Die prozentuale Hemmung der Leukozyten-Migration nach Gabe der Prüfsubstanzen wurde im Vergleich zur Kontrolle ermittelt. Die mittleren Hemmdosen ($ED_{50}$) wurden anhand der Regressionsgeraden berechnet.

2.2 Hemmwirkung

Tabelle 2

| Beispiel Nr. | Dosis (ig) | Hemmung der Leukozyten-Migration (Kontrolle = 0%) |
|---|---|---|
| 1 | 50 mg/kg | 25% |
| 3 | 50 mg/kg | 50% |

3. Hemmung der PGE-2- und LTC-4-Synthese und -Freisetzung

3.1. Methode

Die In-vitro-Wirksamkeit der erfindungsgemässen Verbindungen wird nach an sich bekannten Methoden nachgewiesen (vgl. Brune, K. u.a., Nature 274, 262 (1978); Brune, K. u.a., Naunyn Schmiedeberg's Arch. Pharmacol. 315, 269 (1981); Peskar, B.A. u.a. (1979) in «Radioimmunoassay of drugs and hormones in cardiovascular medicine», Eds. Albertini, A., Da Prada, M., Peskar, B.A., Elsevier, Amsterdam).

3.2 Hemmwirkung

| | $PGE_2$ (% von Kontrollen bei) | | | $LTC_4$ (% von Kontrollen bei) | | |
|---|---|---|---|---|---|---|
| | $10^{-4}$ | $10^{-5}$ | $10^{-6}$ | $10^{-4}$ | $10^{-5}$ | $10^{-6}$ |
| Beispiel 1 | 29 | 84 | 99 | 51 | >100 | >100 |

Beispiel 1 zeigt einen Cyclooxygenasehemmer mit Lipoxygenasehemmer bei $10^{-4}$.

Die übrigen Beispiele zeigen ähnliche pharmakologische Wirkungen.

Beispiel 1

Herstellung von $N^1$-(Benzothiazol-2-yl)-$N^3$, $N^3$-dimethylformamidrazon

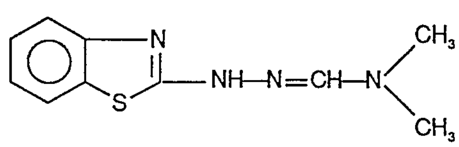

15,28 g (92,5 mmol) 2-Hydrazino-benzothiazol und 40,80 g (277,5 mmol) N,N-Dimethylformamid-diethylacetal werden unter Stickstoffatmosphäre 2 Stunden auf 110°C erhitzt. Der ausgefallene Feststoff wird abgesaugt und aus Isopropanol umkristallisiert.

Farblose Kristalle vom Schmelzpunkt 170-172°C

Ausbeute: 12,55 g (62% d.Th.)

Rf = 0,62 ($CH_2Cl_2$/MeOH 90:10)

$C_{10}H_{12}N_4S$ (220)

$^1$H-NMR-Spektrum: (d$_6$-DMSO, TMS als interner Standard)

δ = 2,87 (s) (-N($C\underline{H}_3)_2$) 6 H,

6,83-7,80 (m) (Aromaten-$\underline{H}$, = C(H)) 5 H,

9,70 (breit) (N-$\underline{H}$) (austauschbar mit $D_2O$) 1 H ppm.

Beispiel 2

a) Herstellung von 2-N¹-Methylhydrazinobenzothiazol

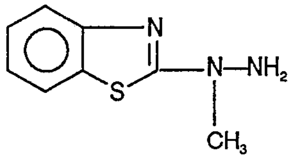

6,78 g (40 mmol) 2-Chlorbenzothiazol werden in 50 ml Ethanol gelöst und mit 3,7 g (80 mmol) Methylhydrazin bei Raumtemperatur 75 Minuten umgesetzt. Nach dem Einengen der Lösung fallen Kristalle an, die mit Wasser digeriert werden. Die Festsubstanz wird abgesaugt und getrocknet.

Farblose Kristalle vom Schmelzpunkt 134–136°C

Ausbeute: 3,6 g (50,2% d.Th.)

Rf = 0,3 ($CH_2Cl_2$/MeOH 95:5)

$C_8H_9N_3S$ (179)

¹H-NMR-Spektrum: ($d_6$-DMSO, TMS als interner Standard)
δ = 3,37 (s) (-N(C<u>H</u>$_3$) 3 H,
5,43–7,80 (s) (-N<u>H</u>$_2$) (austauschbar mit $D_2O$) 2 H,
6,87–7,80 (m) (Aromaten-<u>H</u>) 4 H ppm.

b) Herstellung von N¹-(Benzothiazol-2-yl)-N¹, N³, N³-trimethylformamidrazon

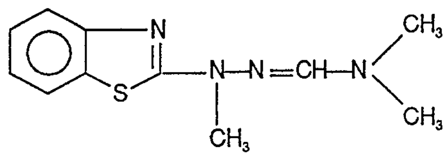

Die Umsetzung erfolgt analog Beispiel 1 mit 1,16 g (6,5 mmol) 2-N¹-Methylhydrazinobenzothiazol und 2,86 g (19,4 mmol) Dimethylformamid-diethylacetal.

Farblose Kristalle vom Schmelzpunkt 87–88°C
Ausbeute: 0,62 g (41% d.Th.)
Rf = 0,58 ($CH_2Cl_2$/MeOH 90:10)

$C_{11}H_{14}N_4S$ (234)

¹H-NMR-Spektrum: ($CDCl_3$, TMS als interner Standard)
δ = 2,90 (s) (-N(C<u>H</u>$_3$)$_2$) 6 H,
3,50 (s) (-C<u>H</u>$_3$) 3 H,
6,90–7,70 (m) (Aromaten-<u>H</u>, = C–<u>H</u>) 5 H ppm.

Beispiel 3

Herstellung von N²-(Benzothiazol-2-yl)-N²-methyl-formhydrazid

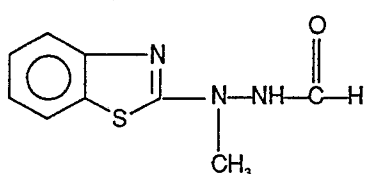

Eine Lösung aus 1 g (5,6 mmol) 2-N¹-Methylhydrazino-benzothiazol, 1 g (21,7 mmol) Ameisensäure und 3 ml $H_2O$ wird 1 Std. auf 100°C erhitzt. Nach dem Abkühlen der Reaktionslösung werden die anfallenden farblosen Kristalle abgesaugt.

Farblose Kristalle vom Schmelzpunkt 148–149°C

Ausbeute: 0,81 g (70% d.Th.)

Rf = 0,50 ($CH_2Cl_2$/MeOH 90:10)

$C_9H_9N_3OS$ (207)

¹H-NMR-Spektrum: ($d_6$-DMSO, TMS als interner Standard)
δ = 3,38 (s) (-C<u>H</u>$_3$) 3 H,
7,07–8,00 (m) (Aromaten-<u>H</u>) 4 H,
8,30 (s) (O=C-<u>H</u>) 1 H,
10,73 (s) (N-<u>H</u>) (austauschbar mit $D_2O$) 1 H ppm.

**Patentansprüche für die Vertragsstaaten BE, CH, DE, FR, GB, IT, LI, LU, NL, SE**

1. Benzothiazolderivat der allgemeinen Formel I

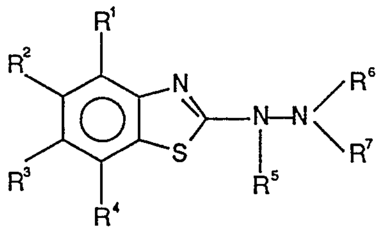
(I)

in der $R^1$, $R^2$, $R^3$ und $R^4$ jeweils ein Wasserstoffatom bedeuten, $R^5$ für ein Wasserstoffatom oder eine Methylgruppe steht, $R^6$ ein Wasserstoffatom und $R^7$ eine Formylgruppe bedeuten oder $R^6$ und $R^7$ für die Gruppierung stehen.

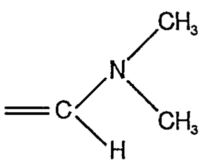

sowie die physiologisch verträglichen Salze davon.

2. $N^1$-(Benzothiazol-2-yl)-$N^3$,$N^3$-dimethylformamidrazon und die physiologisch verträglichen Salze davon.

3. $N^1$-(Benzothiazol-2-yl)-$N^1$,$N^3$,$N^3$-trimethylformamidrazon und die physiologisch verträglichen Salze davon.

4. $N^2$-(Benzothiazol-2-yl)-$N^2$-methylformhydrazid und die physiologisch verträglichen Salze davon.

5. Verfahren zur Herstellung der Benzothiazolderivate nach den Ansprüchen 1 bis 4, dadurch gekennzeichnet, dass man
a) ein Benzothiazolderivat der allgemeinen Formel II

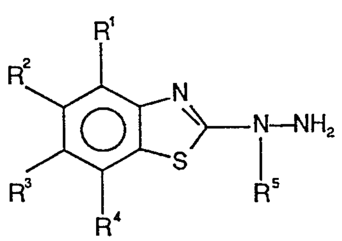
(II)

in der $R^1$ bis $R^5$ die in Anspruch 1 angegebenen Bedeutungen besitzen, mit Ameisensäure zu einer erfindungsgemässen Verbindung der allgemeinen Formel I, in der $R^6$ ein Wasserstoffatom und $R^7$ eine Formylgruppe bedeutet, umsetzt oder
b) ein Benzothiazolderivat der allgemeinen Formel II

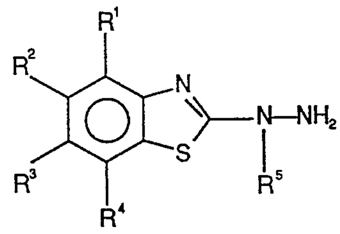
(II)

in der $R^1$ bis $R^5$ die in Anspruch 1 angegebenen Bedeutungen besitzen, mit einem Dialkylaminoacetal der allgemeinen Formel VIII

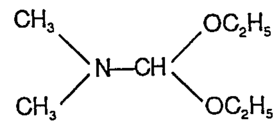
(VIII)

zu einer erfindungsgemässen Verbindung der allgemeinen Formel I, in der $R^6$ und $R^7$ für die Gruppierung

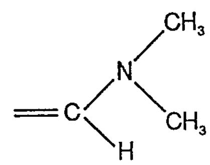

stehen, umsetzt und die erhaltenen Derivate gegebenenfalls in ihre physiologisch verträglichen Salze umwandelt.

6. Arzneimittel, dadurch gekennzeichnet, dass es eine Verbindung nach einem der Ansprüche 1 bis 4 zusammen mit mindestens einem inerten, pharmazeutisch annehmbaren Träger oder Verdünnungsmittel enthält.

**Patentansprüche für den Vertragsstaat AT**

1. Verfahren zur Herstellung von Benzothiazolderivaten der allgemeinen Formel I

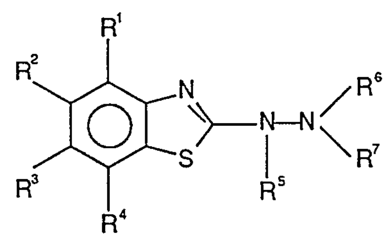
(I)

in der $R^1$, $R^2$, $R^3$ und $R^4$ jeweils ein Wasserstoffatom bedeuten, $R^5$ für ein Wasserstoffatom oder eine Methylgruppe steht, $R^6$ ein Wasserstoffatom und $R^7$ eine Formylgruppe bedeuten oder $R^6$ und $R^7$ für die Gruppierung

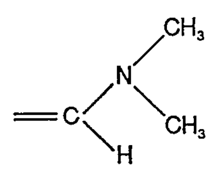

stehen, sowie der physiologisch verträglichen Salze davon, dadurch gekennzeichnet, dass man

a) ein Benzothiazolderivat der allgemeinen Formel II

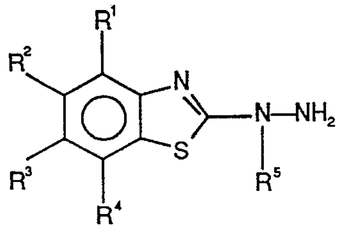

(II)

in der $R^1$ bis $R^5$ die oben angegebenen Bedeutungen besitzen, mit Ameisensäure zu einer erfindungsgemässen Verbindung der allgemeinen Formel I, in der $R^6$ ein Wasserstoffatom und $R^7$ eine Formylgruppe bedeutet, umsetzt oder

b) ein Benzothiazolderivat der allgemeinen Formel II

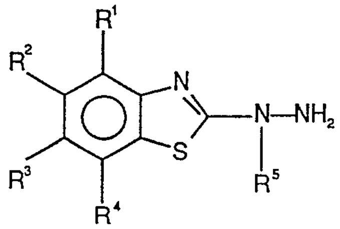

(II)

in der $R^1$ bis $R^5$ die oben angegebenen Bedeutungen besitzen, mit einem Dialkylaminoacetal der allgemeinen Formel VIII

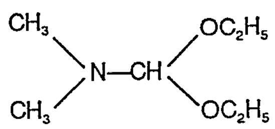

(VIII)

zu einer erfindungsgemässen Verbindung der allgemeinen Formel I, in der $R^6$ und $R^7$ für die Gruppierung

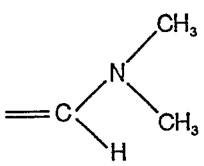

stehen, umsetzt und die erhaltenen Derivate gegebenenfalls in ihre physiologisch verträglichen Salze umwandelt.

2. Verfahren nach Anspruch 1 zur Herstellung von $N^1$-(Benzothiazol-2-yl)-$N^3$,$N^3$-dimethylformamidrazon und der physiologisch verträglichen Salze davon.

3. Verfahren nach Anspruch 1 zur Herstellung von $N^1$-(Benzothiazol-2-yl)-$N^1$,$N^3$,$N^3$-trimethylformamidrazon und der physiologisch verträglichen Salze davon.

4. Verfahren nach Anspruch 1 zur Herstellung von $N^2$-(Benzothiazol-2-yl)-$N^2$-methylformhydrazid und der physiologisch verträglichen Salze davon.

**Claims for the Contracting States BE, CH, DE, FR, GB, IT, LI, LU, NL, SE**

1. Benzothiazole derivative corresponding to the general formula I

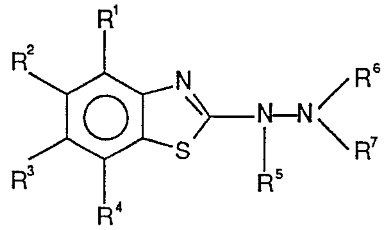

(I)

wherein $R^1$, $R^2$, $R^3$ and $R^4$ each denotes a hydrogen atom, $R^5$ denotes a hydrogem atom or a methyl group, $R^6$ denotes a hydrogen atom and $R^7$ denotes a formyl group or $R^6$ and $R^7$ together stand for the group of the following formula

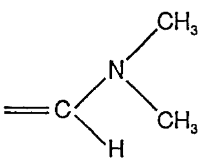

and the physiologically acceptable salts thereof.

2. $N^1$-(Benzothiazol-2-yl)-$N^3$,$N^3$-dimethylformamidrazone and the physiologically acceptable salts thereof.

3. $N^1$-(Benzothiazol-2-yl)-$N^1$,$N^3$,$N^3$-trimethylformamidrazone and the physiologically acceptable salts thereof.

4. $N^2$-(Benzothiazol-2-yl)-$N^2$-methylformhydrazide and the physiologically acceptable salts thereof.

5. Process for the preparation of the benzothiazole derivatives according to claims 1 to 4, characterised in that

a) a benzothiazole derivative corresponding to the general formula II

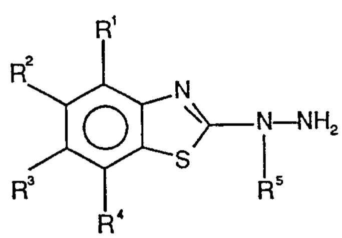

(II)

wherein $R^1$ to $R^5$ have the meanings indicated in claim 1 is reacted with formic acid to form a compound according to the invention corresponding to the general formula I in which $R^6$ denotes a hydrogen atom and $R^7$ a formyl group or

b) a benzothiazole derivative corresponding to the general formula II

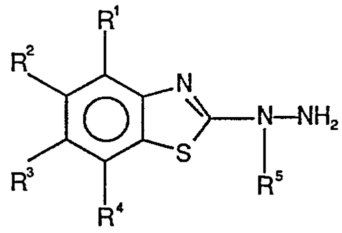

(II)

wherein $R^1$ to $R^5$ have the meanings indicated in claim 1 is reacted with a dialkylaminoacetal corresponding to the general formula VIII

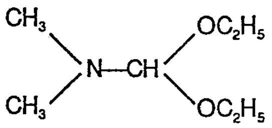

(VIII)

to form a compound according to the invention corresponding to the general formula I in which $R^6$ and $R^7$ stand for the group corresponding to formula:

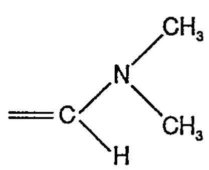

and the derivatives obtained are optionally converted into their physiologically acceptable salts.

6. Pharmaceutical composition, characterised in that it contains a compound according to one of the claims 1 to 4 together with at least one inert, pharmaceutically acceptable carrier or diluent.

**Claims for the Contracting State AT**

1. Process for the preparation of benzothiazole derivatives corresponding to the general formula

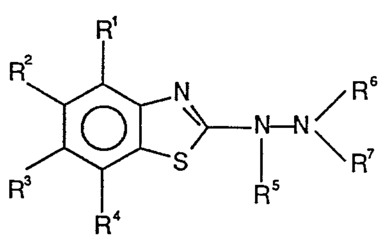

(I)

wherein $R^1$, $R^2$, $R^3$ and $R^4$ each denotes a hydrogen atom, $R^5$ denotes a hydrogen atom or a methyl group, $R^6$ denotes a hydrogen atom and $R^7$ denotes a formyl group or $R^6$ and $R^7$ stand for the group of the formula:

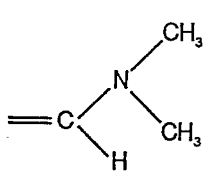

and the physiologically acceptable salts thereof, characterised in that

a) a benzothiazole derivative corresponding to the general formula II

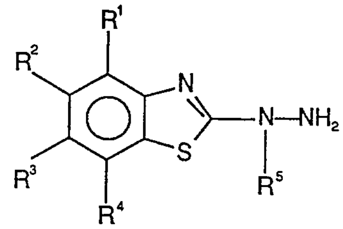

(II)

wherein $R^1$ to $R^5$ have the meanings indicated above is reacted with formic acid to form a compound according to the invention corresponding to the general formula I wherein $R^6$ denotes a hydrogen atom and $R^7$ a formyl group, or

b) a benzothiazole derivative corresponding to the general formula (II)

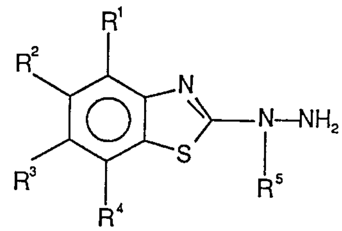

(II)

wherein $R^1$ to $R^5$ have the meanings indicated above is reacted with a dialkylaminoacetal corresponding to the general formula (VIII)

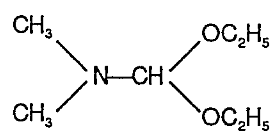

(VIII)

to form a compound according to the invention corresponding to the general formula I in which $R^6$ and $R^7$ stand for the group of the formula

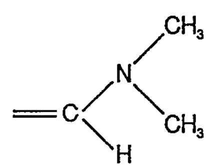

and the derivatives obtained are optionally converted into their physiologically acceptable salts.

2. Process according to claim 1 for the preparation of $N^1$-(benzothiazol-2-yl)-$N^3$,$N^3$-dimethylformamidrazone and the physiologically acceptable salts thereof.

3. Process according to claim 1 for the preparation of $N^1$-(benzothiazol-2-yl)-$N^1$,$N^3$,$N^3$-trimethylformamidrazone and the physiologically acceptable salts thereof.

4. Process according to claim 1 for the preparation of $N^2$-(benzothiazol-2-yl)-$N^2$-methylformhydrazide and the physiologically acceptable salts thereof.

**Revendications pour les Etats contractants: BE, CH, DE, FR, GB, IT, LI, LU, NL, SE**

1. Dérivés du benzothiazole répondant à la formule générale I

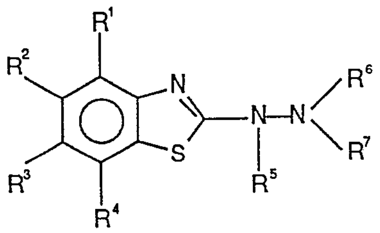

(I)

dans laquelle $R^1$, $R^2$, $R^3$ et $R^4$ représentent chacun un atome d'hydrogène, $R^5$ représente un atome d'hydrogène ou un groupe méthyle, $R^6$ représente un atome d'hydrogène et $R^7$ représente un groupe formyle, ou bien $R^6$ et $R^7$ représentent le groupement

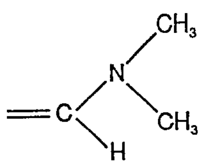

ainsi que leurs sels physiologiquement compatibles.

2. $N^1$-(benzothiazol-2-yl)-$N^3$, $N^3$-diméthylformamidrazone et ses sels physiologiquement compatibles.

3. $N^1$-(benzothiazol-2-yl)-$N^1$,$N^3$,$N^3$-triméthylformamidrazone et ses sels physiologiquement compatibles.

4. $N^2$-(benzothiazol-2-yl)-$N^2$-méthylformhydrazide et ses sels physiologiquement compatibles.

5. Procédé de préparation des dérivés du benzothiazole selon les revendications 1 à 4, caractérisé en ce que:

a) on fait réagir un dérivé du benzothiazole répondant à la formule générale II

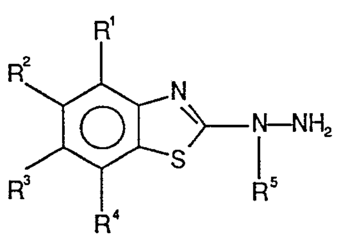

(II)

dans laquelle $R^1$ à $R^5$ ont les significations indiquées dans la revendication 1 sur de l'acide formique pour obtenir un composé selon l'invention répondant à la formule générale I dans laquelle $R^6$ représente un atome d'hydrogène et $R^7$ un groupe formyle, ou

b) on fait réagir un dérivé du benzothiazole répondant à la formule générale II

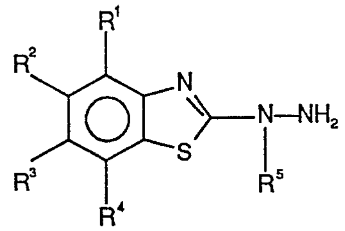

(II)

dans laquelle $R^1$ à $R^5$ ont les significations indiquées dans la revendication 1 sur un dialkylaminoacétal répondant à la formule générale VIII

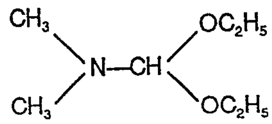

(VIII)

pour obtenir un composé selon l'invention répondant à la formule générale I dans laquelle $R^6$ et $R^7$ représentent le groupement

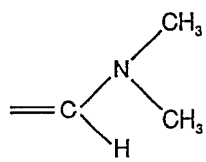

et l'on transforme éventuellement les dérivés obtenus en leurs sels physiologiquement compatibles.

6. Médicament, caractérisé en ce qu'il renferme un composé selon l'une des revendications 1 à 4, avec au moins un support ou diluant inerte pharmaceutiquement acceptable.

**Revendications pour l'Etat contractant: AT**

1. Procédé de préparation de dérivés du benzothiazole répondant à la formule générale I

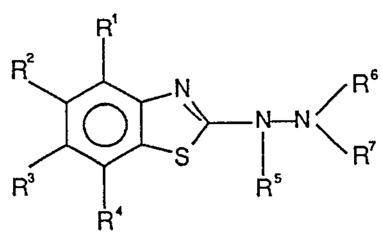

(I)

dans laquelle $R^1$, $R^2$, $R^3$ et $R^4$ représentent chacun un atome d'hydrogène, $R^5$ représente un atome d'hydrogène ou un groupe méthyle, $R^6$ représente un atome d'hydrogène et $R^7$ représente un groupe formyle, ou bien $R^6$ et $R^7$ représentent le groupement

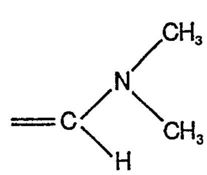

ainsi que leurs sels physiologiquement compatibles, caractérisé en ce que:
a) on fait réagir un dérivé du benzothiazole répondant à la formule générale II

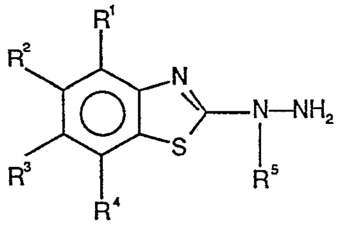

(II)

dans laquelle $R^1$ à $R^5$ ont les significations indiquées précédemment sur de l'acide formique pour obtenir un composé selon l'invention répondant à la formule générale I dans laquelle $R^6$ représente un atome d'hydrogène et $R^7$ un groupe formyle, ou
b) on fait réagir un dérivé du benzothiazole répondant à la formule générale II

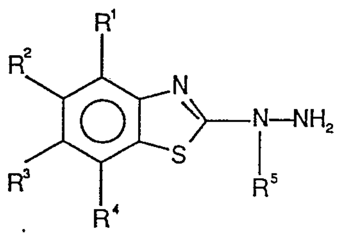

(II)

dans laquelle $R^1$ à $R^5$ ont les significations indiquées précédemment sur un dialkylaminoacétal répondant à la formule générale VIII

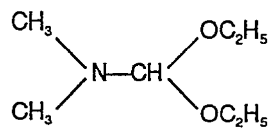

(VIII)

pour obtenir un composé selon l'invention répondant à la formule générale I, dans laquelle $R^6$ et $R^7$ représentent le groupement

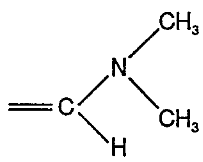

et l'on transforme éventuellement les dérivés obtenus en leurs sels physiologiquement compatibles.

2. Procédé selon la revendication 1 pour préparer la $N^1$-(benzothiazol-2-yl)-$N^3$,$N^3$-diméthylformamidrazone et ses sels physiologiquement compatibles.

3. Procédé selon la revendication 1 pour préparer la $N^1$-(benzothiazol-2-yl)-$N^1$,$N^3$,$N^3$-triméthylformamidrazone et ses sels physiologiquement compatibles.

4. Procédé selon la revendication 1 pour préparer la $N^2$-(benzothiazol-2-yl)-$N^2$-méthylformhydrazide et ses sels physiologiquement compatibles.